(19) Europäisches Patentamt
European Patent Office
Office européen des brevets

(11) Veröffentlichungsnummer : **0 044 421**
**B1**

(12) # EUROPÄISCHE PATENTSCHRIFT

(45) Veröffentlichungstag der Patentschrift : **24.08.83**

(51) Int. Cl.$^3$ : **C 07 D249/12**

(21) Anmeldenummer : **81104774.5**

(22) Anmeldetag : **22.06.81**

(54) **Di- und Oligo-1,2,4-triazolidin-3,5-dione und Verfahren zu ihrer Herstellung.**

(30) Priorität : **21.07.80 DE 3027611**

(43) Veröffentlichungstag der Anmeldung : **27.01.82 Patentblatt 82/04**

(45) Bekanntmachung des Hinweises auf die Patenterteilung : **24.08.83 Patentblatt 83/34**

(84) Benannte Vertragsstaaten : **CH DE FR GB IT LI**

(56) Entgegenhaltungen :
**DE A 2 342 929**
**US A 3 141 023**
**US A 3 663 564**

(73) Patentinhaber : **BAYER AG**
**Zentralbereich Patente, Marken und Lizenzen**
**D-5090 Leverkusen 1, Bayerwerk (DE)**

(72) Erfinder : **Giesecke, Henning, Dr.**
**Kalk-Mülheimer-Strasse 400**
**D-5000 Koeln 80 (DE)**
Erfinder : **Merten, Rudolf, Dr.**
**Berta-von-Suttner-Strasse 55**
**D-5090 Leverkusen (DE)**
Erfinder : **Rottmaier, Ludwig**
**Bergstrasse 85**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 044 421 B1

Di- und Oligo-1,2,4-triazolidin-3,5-dione und Verfahren zu ihrer Herstellung

Die Erfindung betrifft Di- und Oligotriazolidin-3,5-dione sowie Verfahren zu ihrer Herstellung.

Die Herstellung von 1,2,4-Triazolidin-3,5-dion aus Hydrazodicarbonamid und von 4-Phenyl-1,2,4-triazoli din-3,5-dion aus Hydrazodicarbonamid und Anilinhydrochlorid ist bekannt. Sie wird, wie in Liebigs Ann. *283,* 41 (1894) beschrieben, durch Erhitzen von Hydrazodicarbonamid in der Schmelze durchgeführt. Diese Methode ist im technischen Maßstab jedoch kaum durchführbar. Die erhaltene Schmelze ist stark verunreinigt und fällt nach dem Erkalten als feste, harte Masse an, die zerkleinert und gereinigt werden muß. Die Ausbeuten an 1,2,4-Triazolidin-3,5-dion liegen bei 40 bis 50 % der Theorie. Bei der Umsetzung von Hydrazodicarbonamid mit Anilinhydrochlorid entsteht das 4-Phenyl-1,2,4-triazolidin-3,5-dion nur als Nebenprodukt.

Eine andere Herstellungsmethode für 1,2,4-Triazolidin-3,5-dione ist die Cyclisierung von Alkoxycarbonylsemicarbaziden, wie sie im Archiv der Pharmazie *294,* 370 (1961) beschrieben wird. Eine industrielle Verwertbarkeit dieses Verfahrens ist bisher am hohen Preis der Ausgangsverbindungen und wegen der Hydrolyseempfindlichkeit der Zwischenprodukte gescheitert. Alkylen-4,4'-bis-1,2,4-triazolidin-3,5-dione, die allerdings in 1,2-Stellung durch Alkyl oder Aryl substituiert sind, konnten bisher nur durch Alkylierung der entsprechenden in 1,2-Stellung substituierten 1,2,4-Triazolidin-3,5-dione erhalten werden [Archiv der Pharmazie *299,* 81 (1966)]. Dieses Verfahren läßt sich aber nicht auf in 1- und 2-Stellung unsubstituierte Triazolidin-dione anwenden, da diese Stellungen ebenfalls alkyliert werden können.

Aus der US-PS 3 141 023 ist 4-Methylphenyl-1,3-bis-4-urazol als Zwischenprodukt für die Herstellung von 4-Methylphenyl-1,3-bis(1,2-di [N,N-dimethylcarbamyl]-urazol) bekannt, wobei letztere Verbindung auf dem Pflanzenschutzsektor zur Bekämpfung von Nematoden oder aber zur Behandlung von Cellulosegeweben verwendet wird, um deren Waschbarkeit und Knitterfestigkeit zu verbessern. Demgegenüber werden die erfindungsgemäß beanspruchten Bis- und Oligourazole (= Di- und Oligo-1,2,4-triazolidin-3,5-dione) durch Umsetzung mit Alkylenoxiden in Polyhydroxyalkylurazole überführt, die als vernetzende Komponenten in temperaturbeständigen Elektroisolierlacken Verwendung finden, oder mit Epihalogenhydrin in Polyglycidylurazole, die als Vernetzer in Pulverlacken eingesetzt werden.

Gegenstand der Erfindung sind neue Di- und Oligo-1,2,4-triazolidin-3,5-dione der allgemeinen Formel (I),

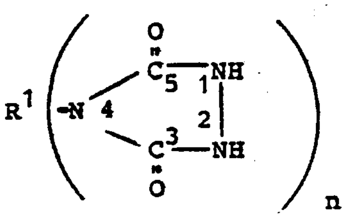

(I)

worin

$R^1$ einen zwei- bis fünfwertigen unsubstituierten oder substituierten, linearen oder verzweigten aliphatischen $C_2$-$C_{30}$, vorzugsweise $C_2$-$C_{12}$, einen zwei- bis fünfwertigen unsubstituierten oder substituierten cycloaliphatischen $C_5$-$C_{21}$, einen zwei- bis fünfwertigen unsubstituierten oder substituierten aliphatisch-aromatischen $C_7$-$C_{17}$, vorzugsweise $C_7$-$C_{10}$, oder einen zwei- bis fünfwertigen unsubstituierten oder substituierten aromatischen $C_6$-$C_{21}$, vorzugsweise $C_6$-$C_{15}$ Rest bedeuten, die vorgenannten substituierten Reste enthalten als Substituenten mindestens eine Alkoxycarbonylgruppe mit $C_1$-$C_4$ in der Alkoxygruppe, CN, $NO_2$, Alkylmercaptogruppe mit $C_1$-$C_4$ in der Alkylgruppe, Dialkylaminogruppe mit $C_1$-$C_6$ in jeder Alkylgruppe und Halogen, die vorgenannten aliphatischen Reste können ferner durch ein oder mehrere Sauerstoffatome oder tertiäre Stickstoffatome, die vorgenannten mehrkernigen aliphatisch-aromatischen, mehrkernigen cycloaliphatischen und mehrkernigen aromatischen Reste durch mindestens eine Alkylengruppe mit 1-4 C-Atomen, durch mindestens ein Sauerstoffatom oder tertiäres Stickstoffatom oder durch mindestens eine Sulfonylgruppe

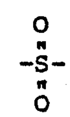

unterbrochen sein, und

n eine Zahl von 2 bis 5, vorzugsweise 2 oder 3, darstellen.

Besonders bevorzugt sind Verbindungen der Formel (I), in denen der Rest $R^1$ unsubstituiert ist. Formelmäßig seien beispielhaft folgende bevorzugte Reste $R^1$ angegeben :

1. $-(CH_2)_n-$ n = 2-20, vorzugsweise 2-12,

2. $-[CH_2-\overset{R^2}{CH}-O]_m-CH_2-\overset{R^2}{CH}-$ $R^2$ = H, $CH_3$ ; m = 1-9

3. $-[CH_2-(CH_2)_q-\overset{R_3}{N}]_p-(CH_2)_q-CH_2-$ $R_3$ = Alkyl $C_1$-$C_4$, p = 1-4, q = 1-2 ;

4. 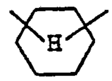

5. 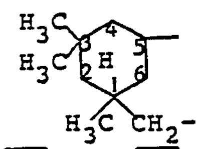

6. 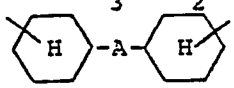 A = Alkylen mit 1-4 C-Atomen, O, —$N(CH_3)$— ;

7. 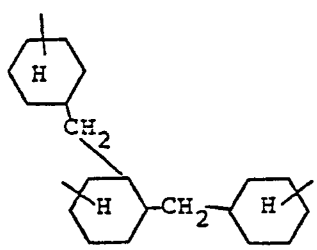

8. 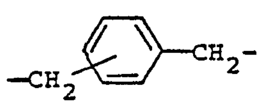

9. 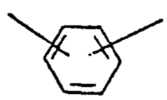

10. 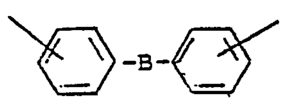 B = Alkylen mit 1-4 C-Atomen, O, —$N(CH_3)$—,

11. 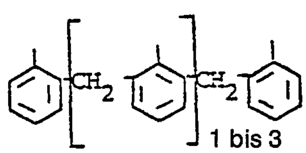
1 bis 3

12. $-(CH_2)_3-O\{CH_2\}_{2\ bis\ 4}-O-(CH_2)_3-$

Die erfindungsgemäßen Di- und Oligo-1,2,4-triazolidin-3,5-dione der allgemeinen Formel (I) können nach verschiedenen Verfahren erhalten werden.

So können Di- und Polyamide der Formel (II),

$$R^1(NH_2)_n \qquad (II)$$

worin $R^1$ und n die für Formel (I) angegebene Bedeutung besitzen, mit Hydrazodicarbonamid (Verfahren 1) oder mit 1,2,4-Triazolidin-3,5-dion (Verfahren 2) bei erhöhten Temperaturen, vorzugsweise in Gegenwart eines Lösungsmittels oder Lösungsmittelgemisches unter Abspaltung von Ammoniak in die Di- und Oligo-1,2,4-triazolidin-3,5-dione der allgemeinen Formel (I) überführt werden. Auf 0,9-1,1 Mol Hydrazodicarbonamid bzw. auf 0,9-1,1 Mol 1,2,4-Triazolidin-3,5-dion werden dabei vorzugsweise 1/m Mole des Di- bzw. Polyamins eingesetzt (m = Anzahl der primären $NH_2$-Gruppen pro Molekül).

Ein weiteres Verfahren (Verfahren 3) besteht darin, daß N-monosubstituierte Hydrazodicarbonamide der Formel (III),

$$[H_2N-\overset{O}{\overset{\|}{C}}-NH-NH-\overset{O}{\overset{\|}{C}}-NH-]_n R^1 \qquad (III)$$

worin $R^1$ und n die in Formel (I) angegebene Bedeutung haben, unter den Bedingungen, wie für Verfahren 1 und 2 angegeben, unter Ammoniakabspaltung zu den Di- und Oligo-1,2,4-triazolidin-3,5-dionen der Formel (I) cyclisiert werden.

Das bei dem Verfahren 1 einzusetzende Hydrazodicarbonamid ist aus der Literatur bekannt und wird bei der Reaktion von 1 Mol Hydrazin mit 2 Mol Harnstoff unter Ammoniakabspaltung in wäßrigem Medium in praktisch quantitativer Ausbeute erhalten. Das dabei als Niederschlag anfallende Hydrazodicarbonamid wird durch Absaugen isoliert und kann als nutschenfeuchte Ware sofort weiterverarbeitet werden, wenn das Restwasser beim Cyclisierungsprozeß abgeführt werden kann. Selbstverständlich kann auch getrocknetes Hydrazodicarbonamid zur weiteren Reaktion verwendet werden. Es ist auch möglich, aus der erhaltenen Suspension aus Hydrazodicarbonamid in Wasser nach Zusatz eines geeigneten Lösungsmittels durch Erhitzen das Wasser abzudestillieren und das verbleibende Hydrazodicarbonamid mit Aminen zu den erfindungsgemäßen 1,2,4-Triazolidin-3,5-dionen der Formel (I) umzusetzen.

Das bei dem Verfahren 2 einzusetzende Triazolidin-3,5-dion ist ebenfalls aus der Literatur bekannt (vgl. Liebigs Ann. *283,* 41 (1894).

Die für das Verfahren 3 einzusetzenden N-monosubstituierten Hydrazodicarbonamide der Formel (III) werden durch Umsetzung von Semicarbazid mit Isocyanaten der Formel (IV),

$$R^1(NCO)_n \qquad (IV)$$

erhalten,
worin $R^1$ und n die für Formel (I) angegebene Bedeutung besitzen.

Im allgemeinen ist es zweckmäßig, die Umsetzung von Semicarbazid und Isocyanat zu den monosubstituierten Hydrazodicarbonamiden der Formel (III) in einem Lösungs- oder Verdünnungsmittel durchzuführen, wobei dann die Ausgangsmaterialien im wesentlichen in äquivalenten Mengenverhältnissen (1 Mol Semicarbazid = 1 NCO-Gruppe), sowohl gelöst als auch nur suspendiert sein können. Selbstverständlich ist es ebenso möglich, die Umsetzung ohne Anwesenheit eines Lösungs- oder Verdünnungsmittels durchzuführen. Für das Verfahren geeignete Lösungsmittel sind z. B. aromatische Kohlenwasserstoffe, chlorierte aromatische Kohlenwasserstoffe, Benzonitril, aliphatische Kohlenwasserstoffe, Ester und Ketone. Besonders geeignet sind Toluol, Xylol, Mesitylen, Chlorbenzol, Dichlorbenzol, N-Methylpyrrolidon, Dimethylformamid, Dimethylacetamid, Hexamethylphosphorsäuretriamid, Tetramethylharnstoff, Nitromethan und Nitrobenzol; es ist aber auch möglich, die Reaktion bei niedrigen Temperaturen in Wasser oder niederen Alkoholen durchzuführen.

Die Reaktion kann bei − 30 bis 150 °C durchgeführt werden. Bevorzugt arbeitet man bei − 20 bis 100 °C, besonders bevorzugt bei − 10 bis 80 °C.

Es sei dahingestellt, nach welchem Reaktionsmechanismus die drei Verfahren im einzelnen ablaufen. Alle drei Verfahren führen in einfacher, reproduzierbarer Weise und in guten Ausbeuten zu den Di- und Oligo-1,2,4-triazolidin-3,5-dionen der Formel (I). Denkbar ist, daß beim Verfahren 1 aus etwa 1 Mol Hydrazodicarbonamid und 1/m Mol Amin (m = Anzahl der primären Aminogruppen pro Mol Amin) unter Abspaltung von 1 Mol Ammoniak bzw. bei Verfahren 2 aus etwa 1 Mol 1,2,4-Triazolidin-3,5-dion mit 1/m Mol Amin unter Ringöffnung zunächst N-monosubstituiertes Hydrazodicarbonamid der Formel (III) gebildet wird, aus dem unter Abspaltung von Ammoniak unter Cyclisierung die Verbindungen der Formel (I) erhalten werden.

Bei allen drei Verfahren zur Herstellung der Verbindungen der Formel (I) liegt die Reaktionstemperatur im allgemeinen zwischen 150 und 280 °C, vorzugsweise zwischen 170 und 250 °C, und insbesondere zwischen 175 und 220 °C. Je jöher die Temperatur ist, desto schneller verläuft die Reaktion, allerdings

nimmt die Gefahr der Bildung unerwünschter Nebenprodukte sowie die Zersetzung des Lösungsmittels zu.

Die Reaktionszeiten liegen im allgemeinen zwischen 1 und 40 Stunden, können jedoch in Ausnahmefällen auch darüber oder darunter liegen.

Zur Beschleunigung der Reaktion kann es zweckmäßig sein, saure oder basische Katalysatoren zuzugeben. Besonders geeignet sind Metallalkoholate (z. B. Natriummethylat, Zinn(II)octoat) und tert. Amine.

Der Reaktionsdruck liegt normalerweise bei 50 mbar bis 5 bar, wobei bei höherem Druck als bei atmosphärischem Druck zwischendurch das entstehende Ammoniak abgelassen werden muß, so daß die Cyclisierung vorzugsweise bei 300 mbar bis 2 bar Druck durchgeführt wird.

Es kann von Vorteil sein, wenn die Konzentration an abgespaltenem Ammoniak im Reaktionsgefäß niedrig gehalten wird. Dies kann auf jede bekannte Weise erfolgen, z. B. durch Ausblasen mit einem inerten Gas wie Luft, Stickstoff, Kohlendioxid oder Wasserdampf. Auch können niedrigsiedende Lösungsmittel, z. B. aliphatische, aromatische, araliphatische Kohlenwasserstoffe, deren technische Mischungen und chlorierte Kohlenwasserstoffe mit vorzugsweise 1-10 Kohlenstoffatomen wie Cyclohexan, Toluol, Xylole, Petrolether oder Chloroform, die flüssig in den Reaktor gepumpt oder getropft werden, zum Austreiben des Ammoniaks verwendet werden. Der Partialdruck des Ammoniaks kann auch durch Absaugen herabgesetzt werden, indem man bei unteratmosphärischem Druck arbeitet.

Die bei der Umsetzung zu verwendenden organischen Lösungsmittel sollen unter Reaktionsbedingungen eine ausreichende thermische Stabilität aufweisen und chemisch inert gegen Hydrazodicarbonamide bzw. Triazolidin-3,5-dione sein, auch soll der Siedepunkt genügend hoch liegen, damit das Lösungsmittel während der Reaktion nicht abdestilliert.

Die Siedepunkte der Lösungsmittel liegen in der Regel bei Atmosphärendruck bei mindestens 150 °C, vorzugsweise bei ca. 175 bis 280 °C. Die Lösungsmittel können bei Raumtemperatur mit Wasser mischbar, zum Teil mischbar oder nicht mischbar sein.

Geeignete Lösungsmittel sind

A) mit Phenyl oder $C_1$-$C_8$-Alkylgruppen, N-substituierte stickstoffhaltige Lösungsmittel, zum Beispiel N-substituierte Pyrrolidone, Urethane, cyclische Urethane oder Harnstoffe, etwa N-Methylpyrrolidon, Ethyl-phenyl-urethan, 5-Methyl-2-oxazolidon, Tetramethylharnstoff ; weiter Polyether, z. B. Diethylenglykoldiethylether, Phenole wie Kresole, halogensubstituierte Phenole und Kresole, z. B. 4-Chlorphenol ; Dialkylsulfone und cyclische Sulfone jeweils mit maximal 12 Kohlenstoffatomen, z. B. Dimethylsulfon oder Sulfolan ; aromatische oder araliphatische Ether wie Diphenylether und Dibenzylether, hochsiedende Alkohole, z. B. Ethylenglykol.

Geeignete Lösungsmittel sind ferner

B) aliphatische, cycloaliphatische, aromatische und araliphatische Kohlenwasserstoffe sowie deren technische Gemische wie z. B. Dodecan, Decalin, Trimethylbenzol, Naphthalin, 1-Methyl-naphthalin, Diphenylmethan, halogenierte aliphatische, cycloaliphatische, aromatische und araliphatische Kohlenwasserstoffe sowie deren technische Gemische wie Dodecylchlorid, 1,2,4-Trichlorbenzol, 1-Chlornaphthalin, Dichlor-toluol.

Besonders bevorzugt sind Diphenylether, Diphenylmethan, 1-Methylnaphthalin, Dialkylsulfone und cyclische Sulfone, insbesondere Sulfolan und N-Methylpyrrolidon.

Die mit den polaren Lösungsmitteln, wie vorstehend unter A) aufgeführt, nach der Cyclisierung (Umsetzung) erhaltenen Reaktionsmischungen können während des Abkühlungsvorgangs mit gegen Triazolidin-3,5-dione inerten Lösungsmitteln wie aliphatischen, cycloaliphatischen, araliphatischen oder aromatischen Kohlenwasserstoffen, z. B. Cyclohexan, Toluol, Xylol, aliphatischen oder cycloaliphatischen Alkoholen, z. B. Butanol, Cyclohexanol und daraus abgeleiteten Ethern oder Estern, z. B. Glykolmonomethylether, Essigsäurebutylester, Ketone, z. B. Aceton oder Ethylmethylketon und bei Verwendung von mit Wasser mischbaren polaren Lösungsmitteln auch Wasser, abgemischt werden, so daß die auskristallisierenden Triazolidin-3,5-dione in größeren Ausbeuten bzw. höheren Reinheiten anfallen. Die Zusätze betragen bis zu 500 Gew.-%, bezogen auf polares Lösungsmittel.

Die abgehandelten Verfahren sind sowohl für diskontinuierliche als auch für kontinuierliche Arbeitsweise geeignet. Bei der kontinuierlichen Arbeitsweise wird die Cyclisierung nach bekannten Verfahren, z. B. durch die Benutzung von Kaskaden oder der Verwendung von Röhrenreaktoren, durchgeführt. Die diskontinuierliche Verfahrensweise ist bevorzugt.

Die glatte Reaktion des abgehandelten Verfahrens 1 war überraschend, da zu erwarten war, daß bei der Cyclisierung von Hydrazodicarbonamid in Gegenwart von primären Aminen schwer trennbare Gemische von 1,2,4-Triazolidin-3,5-dion und Di- und Oligo-1,2,4-triazolidin-3,5-dionen der Formel (I) entstehen würden.

Es war ferner überraschend, daß das 1,2,4-Triazolidin-3,5-dion (Verfahren 2) mit primären Aminen zu 4-substituierten Triazolidin-3,5-dionen reagiert, obwohl es thermisch sehr beständig ist.

Die Di- und Oligo-1,2,4-triazolidin-3,5-dione der Formel (I) sind wertvolle Ausgangsstoffe zur Herstellung von temperaturbeständigen Polymeren. Hieraus hergestellte Polyhydroxyalkyl-triazolidin-3,5-dione z. B. finden als vernetzende Komponenten in temperaturbeständigen Elektroisolierlacken Verwendung, während entsprechende Polyglycidyl-triazolidin-3,5-dione z. B. als Vernetzer in Pulverlacken, die nach dem elektrostatischen Pulversprühverfahren angewendet werden, eingesetzt werden. Ferner

können Di- und Oligo-1,2,4-triazolidin-3,5-dione der Formel (I) in photographischen Zusammensetzungen eingesetzt werden.

Die in den Beispielen angegebenen Prozente beziehen sich auf das Gewicht.

Beispiel 1

60 g Hydrazodicarbonamid und 29 g 1,6-Diaminohexan werden in 100 ml Sulfolan 2 Stunden bei 175 °C und 9 Stunden bei 200 °C gerührt. Beim Abkühlen fällt ein Niederschlag aus, der abgesaugt und aus Wasser umkristallisiert wird. Man erhält 36 g (51 % d. Th.) 1,6-Hexandiyl-4,4'-bis-1,2,4-triazolidin-3,5-dion als farblose Kristalle vom Fp. 215-217 °C.

| | | C | H | N |
|---|---|---|---|---|
| $C_{10}H_{16}N_6O_4$ | ber. | 42,25 | 5,67 | 29,51 |
| (284,3) | gef. | 42,5 | 5,7 | 29,2 |

Beispiel 2

600 g Hydrazodicarbonamid und 150 g Ethylendiamin werden in 500 ml N-Methylpyrrolidon 4 Stunden bei 175 °C und 20 Stunden bei 200 °C gerührt. Beim Abkühlen fällt ein Niederschlag aus, der abgesaugt und mit Ethanol gewaschen wird. Man erhält 462 g (80 % d. Th.) 1,2-Ethandiyl-4,4'-bis-1,2,4-triazolidin-3,5-dion als farblose Kristalle vom Fp. > 330 °C.

| | | C | H | N |
|---|---|---|---|---|
| $C_6H_8N_6O_4$ | ber. | 31,58 | 3,53 | 36,83 |
| (228,2) | gef. | 31,4 | 3,6 | 36,8 |

IR (KBr) : 1 731, 1 673 $cm^{-1}$ (C = 0).

Beispiel 3

765 g 1-Aminomethyl-5-amino-1,3,3-trimethylcyclohexan und 708 g Hydrazodicarbonamid werden in 1 l N-Methylpyrrolidon 4 Stunden bei 175 °C, 5 Stunden bei 200 °C und 15 Stunden bei 220 °C gerührt. Anschließend wird das Lösungsmittel im Vakuum abdestilliert und der Rückstand im Vakuum getrocknet. Man erhält 1 473 g (97 % d. Th.) 1-(3,5-Dioxo-1,2,4-triazolidin-4-yl-methyl)-1,3,3-trimethyl-5-(3,5-dioxo-1,2,4-triazolidin-4-yl)-cyclohexan ( = Formel I) n = 2, $R^1$ = Rest Nr. 5 der bevorzugt angegebenen Reste $R^1$) als farblose Kristalle vom Fp. 213 °C (Zersetzung).

IR (KBr) : 1 776, 1 714, 1 703 $cm^{-1}$ (C = 0) :

MS (m/e) : Molpeak 338.

Beispiel 4

32 g 2-(4-Amino-cyclohexylmethyl)-4,4'-diamino-dicyclohexylmethan und 36 g Hydrazodicarbonamid werden in 50 ml N-Methylpyrrolidin 4 Stunden bei 175 °C, 3 Stunden bei 200 °C und 1 Stunde bei 220 °C gerührt. Die Lösung wird abgekühlt und in 1 l $H_2O$ eingerührt. Dabei fällt ein Niederschlag aus, der abgesaugt und mit Wasser gewaschen wird. Man erhält 35 g (52 % d. Th.) 2-[4-(3,5-Dioxo-1,2,4-triazolidin-4-yl)-cyclohexylmethyl]-4,4'-bis-(3,5-dioxo-1,2,4-triazolidin-4-yl)-dicyclohexylmethan als farblose Kristalle vom Fp. 270 °C (Zersetzung).

Beispiel 5

420 g 4,4'-Diaminodicyclohexylmethan und 472 g Hydrazodicarbonamid werden in 750 ml N-Methylpyrrolidon 4 Stunden bei 175 °C, 8 Stunden bei 200 °C und 4 Stunden bei 220 °C gerührt. Das Reaktionsprodukt wird abgekühlt und anschliessend in 3 l Wasser eingerührt. Dabei fällt ein Niederschlag aus, der abgesaugt und mit Wasser gewaschen wird. Man erhält 566 g (75 % d. Th.) an 4,4'-Bis-(1,2,4-triazolidin-3,5-dion-4-yl)-dicyclohexylmethan) als farblose Kristalle vom Fp. 305 °C (Zersetzung).

MS (m/e) : Molpeak 378.

Beispiel 6

Eine Lösung von 446 g Semicarbazidhydrochlorid in 2 l Wasser wird mit $Na_2CO_3$ neutralisiert. Anschließend werden unter Rühren bei Raumtemperatur 250 g Diphenylmethan-4,4'-diisocyanat, gelöst in 400 ml Dioxan, innerhalb von 2 Stunden zugetropft. Man rührt 6 Stunden bei 40-60 °C nach, saugt vom entstandenen Niederschlag ab und wäscht den Niederschlag mit Wasser. Anschließend wird der feuchte Niederschlag in 1 l Sulfolan suspendiert und die Suspension so lange auf 160 °C erwärmt, bis kein Wasser mehr abdestilliert. Dann wird ein Vakuum von 300 mbar angelegt und die Suspension 10 Stunden

bei 200 °C gerührt. Beim Abkühlen entsteht ein Niederschlag, der Abgesaugt und mit Methanol gewaschen wird. Man erhält 178 g 4,4'-Bis-(1,2,4-triazolidin-3,5-dion-4-yl)-diphenylmethan als farblose Kristalle vom Fp. 320 °C.

IR (KBr) : 1 781 (Schulter), 1 713 $cm^{-1}$ (C = 0).

MS (m/e) : Molpeak 366.

Beispiel 7

102 g 1,4-Butandiolbis-(3-aminopropylether) und 120 g Hydrazodicarbonamid werden in 300 ml N-Methylpyrrolidon 1 Stunde bei 150 °C, 2 Stunden bei 175 °C und 5 Stunden bei 200 °C gerührt. Beim Abkühlen fällt ein Niederschalg aus, der abgesaugt und mit 200 ml Acetonitril ausgekocht wird. Als Rückstand erhält man 142 g (76 % d. Th.) 1,4-Butandiol-bis-[3-3,5-dioxo-1,2,4-triazolidin-4-yl)-propylether] als farblose Kristalle vom Fp. 152-154 °C.

| | | C | H | N |
|---|---|---|---|---|
| $C_{14}H_{24}N_6O_6$ | ber. | 45,15 % | 6,50 % | 22,57 % |
| (372,4) | gef. | 45,2 % | 6,6 % | 22,6 % |

IR (KBr) : 1 768, 1 674 $cm^{-1}$ (C = 0).

Beispiel 8

100 g 1,12-Diaminododecan und 120 g Hydrazodicarbonamid werden in 300 ml N-Methylpyrrolidon 1 Stunde bei 175 °C und 4 Stunden bei 200 °C gerührt. Beim Abkühlen fällt ein Niederschlag aus, der abgesaugt und mit 300 ml Acetonitril ausgekocht wird. Man erhält 156 g (85 % d. Th.) 1,12-Dodecan-4,4'-bis-1,2,4-triazolidin-3,5-dion als farblose Kristalle vom Fp. 172-175 °C.

| | | C | H | N |
|---|---|---|---|---|
| $C_{16}H_{28}N_6O_4$ | ber. | 52,16 % | 7,66 % | 22,81 % |
| (368,5) | gef. | 52,2 % | 7,7 % | 22,6 % |

MS (m/e) : Molpeak 368.

Beispiel 9

101 g 1,2,4-Triazolidin-3,5-dion und 57 g 1,4-Diaminocyclohexan werden in 500 ml N-Methylpyrrolidon 4 h auf 175 °C und 30 Stunden auf 200 °C unter Rühren erhitzt. Beim Abkühlen kristallisiert ein Niederschlag aus, der abgesaugt, mit Ethanol gewaschen und getrocknet wird. Man erhält 110 g (78 % d. Th) 1,4-Cyclohexandiyl-bis-(1,2,4-triazolidin-3,5-dion-4-yl) als farblose Kristalle vom Fp. > 300 °C.

| | | C | H | N |
|---|---|---|---|---|
| $C_{10}H_{14}N_6O_4$ | ber. | 42,56 % | 4,96 % | 29,75 % |
| (282,2) | gef. | 42,7 % | 5,1 % | 29,1 % |

MS (m/e) : Molpeak 282.

Beispiel 10

72,5 Bis-(3-aminopropyl)-methylamin und 120 g Hydrazodicarbonamid werden in 300 ml N-Methylpyrrolidon 2 Stunden bei 175 °C und 3 Stunden bei 200 °C gerührt. Das Reaktionsprodukt wird im Vakuum eingeengt, in Wasser gelöst und mit Isopropanol gefällt. Man erhält ein farbloses Produkt, das nicht zur Kristallisation zu bringen war. Zur Charakterisierung und Reinigung wird das Produkt in $H_2O$ gelöst, die Lösung mit konz. HCl auf pH = 2 eingestellt und anschließend im Vakuum eingeengt. Der Rückstand wird aus Ethanol umkristallisiert. Man erhält 112 g (65 % d. Th.) Bis-[3-(3,5-dioxo-1,2,4-triazolidin-4-yl)-propyl]-methylaminhydrochlorid als farblose Kristalle vom Fp. 238 °C.

IR (KBr) : 1 763, 1 698 $cm^{-1}$ (C = 0).

Beispiel 11

Eine Lösung von 89 g Semicarbazidhydrochlorid in 250 ml Wasser wird mit $Na_2CO_3$ neutralisiert. Anschließend werden unter Rühren bei Raumtemperatur 101 g 4,4'-Diisocyanatodiphenylether, in 100 ml Dioxan gelöst, innerhalb von 2 Stunden zugetropft. Man rührt 6 Stunden bei 40-60 °C, saugt den entstandenen Niederschlag ab und wäscht ihn mit Aceton. Der Niederschlag wird über Nacht an der Luft getrocknet und anschließend in 600 ml Sulfolan suspendiert. Die Suspension wird so lange auf 160 °C

erhitzt, bis kein Wasser mehr abdestilliert. Dann wird ein Vakuum von 300 mbar angelegt und 20 Stunden bei 200 °C gerührt.

Das Lösungsmittel wird im Vakuum abdestilliert und der Rückstand aus Acetonitril umkristallisiert.

Man erhält 73,6 g (50 % d. Th.) 4,4'-(Bis-1,2,4-triazolidin-3,5-dion-4-yl)-diphenylether als farblose Kristalle vom Fp. 314-315 °C.

$^1$H-NMR (d-DMSO) : δ = 6,8-7,7 ppm (m, 8H aromat.), 8,7 ppm (s, 4 H).

Beispiel 12

64,2 g Hydrazodicarbonamid und 37 g m-Xylylendiamin in 300 ml N-Methylpyrrolidon werden 4 Stunden auf 175 °C und 30 Stunden auf 200 °C unter Rühren erhitzt. Anschließend wird das Lösungsmittel im Vakuum abdestilliert und der Rückstand aus Chloroform umkristallisiert.

Man erhält 58 g (70 % d. Th.) 1,3-Bis(1,2,4-triazolidin-3,5-dion-4-yl-methyl)-benzol als farblose Kristalle vom Fp. 244-246 °C.

IR (KBr) : 1 770 $cm^{-1}$ (Schulter), 1 700 $cm^{-1}$ (C = 0)

1H-NMR (d-DMSO) : δ = 4,5 ppm (s, 2 $CH_2$), 7,2 ppm (s, 4H aromat.), 10,2 ppm (s, 4 H).

Anwendungsbeispiel

388 g Terephthalsäuredimethylester, 202 g 1,2-Ethandiyl-4,4'-bis-[bis-(2-hydroxyethyl)-1,2,4-triazolidin-3,5-dion] und 62 g 1,2-Ethandiol werden aufgeschmolzen, mit 2 g Bleiacetat, 1 g Titantetrabutylat und 50 ml Xylol versetzt und innerhalb von 8 Stunden auf 190 °C aufgeheitzt. Dann wird in 6 Stunden unter leichtem Vakuum auf 220 °C erwärmt und während 3 Stunden bei 220 °C bis 230 °C und verstärktem Vakuum auskondensiert. Es werden 518 g eines braunen, bei Raumtemperatur spröden Harzes erhalten.

35 g dieses Harzes werden in 65 g m-Kresol gelöst, mit 1,6 g einer stabilisierten Titantetrabutylatlösung (hergestellt durch Erwärmen von 1 Gew.-Teil Titantetrabutylat und 2 Gew.-Tlen. Kresol) versetzt, auf ein entfettetes Eisenblech gestrichen, 20 Minuten bei 200 °C und 10 Minuten bei 300 °C eingebrannt. Es wird ein elastischer gut haftender Film von glatter Oberfläche erhalten, der beim Knicken des Eisenbleches nicht reißt und nicht abblättert.

Das für die Herstellung des Polyesters eingesetzte 1,2-Ethandiyl-4,4'-bis-[bis-(2-hydroxyethyl)-1,2,4-triazolidin-3,5-dion] wird wie folgt erhalten :

Zu einer Suspension von 228 g 1,2-Ethandiyl-4,4'-bis-1,2,4-triazolidin-3,5-dion und 2 g Triethylamin in 1 kg Dimethylformamid werden bei 125 °C innerhalb von 6 Stunden 176 g Ethylenoxid so eingeleitet, daß kein Ethylenoxid entweicht. Durch Anlegen von Vakuum wird bei 40 mbar der größte Teil des Lösungsmittels entfernt. Der erhaltene Rückstand von 483 g wird in 500 ml eines Lösungsmittelgemisches aus 7 vol.-Teilen Aceton und 3 Vol.-Teilen Isopropanol unter Erwärmen gelöst. Beim Abkühlen fällt ein Niederschlag aus, der abgesaugt und im Vakuum getrocknet wird.

Man erhält 330 g 1,2-Ethandiyl-4,4'-bis-[bis-(2-hydroxy-ethyl)-1,2,4-triazolidin-3,5-dion] als farblose Kristalle vom Fp. 153-155 °C.

| | | C | H | N |
|---|---|---|---|---|
| $C_{14}H_{24}N_6O_8$ | ber. | 41,58 | 5,98 | 20,78 |
| (404,4) | gef. | 41,4 | 5,7 | 20,9 |

**Ansprüche**

1. Di- und Oligo-1,2,4-triazolidin-3,5-dione der allgemeinen Formel (I),

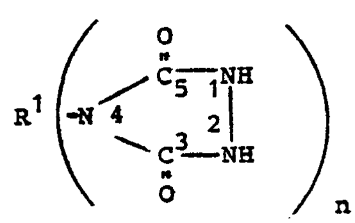

(I)

worin

$R^1$ einen zwei- bis fünfwertigen unsubstitutierten oder substituierten, linearen oder verzweigten aliphatischen $C_2$-$C_{30}$, einen zwei- bis fünfwertigen unsubstituierten oder substituierten cycloaliphatischen $C_5$-$C_{21}$, einen zwei- bis fünfwertigen unsubstituierten oder substituierten aliphatisch-aromatischen $C_7$-$C_{17}$, oder einen zwei- bis fünfwertigen unsubstituierten oder substituierten aromatischen $C_6$-$C_{21}$-Rest bedeuten, die vorgenannten substituierten Reste enthalten als Substituenten mindestens eine Alkoxycarbonylgruppe mit $C_1$-$C_4$ in der Alkoxygruppe, CN, $NO_2$, Alkylmercaptogruppe mit $C_1$-$C_4$ in der Alkylgruppe, Dialkylaminogruppe mit $C_1$-$C_6$ in jeder Alkylgruppe und Halogen, die vorgenannten

aliphatischen Reste können ferner durch ein oder mehrere Sauerstoffatome oder tertiäre Stickstoffatome, die vorgenannten mehrkernigen aliphatisch-aromatischen, mehrkernigen cycloaliphatischen und mehrkernigen aromatischen Reste durch mindestens eine Alkylengruppe mit 1-4 C-Atomen, durch mindestens ein Sauerstoffatom oder tertiäres Stickstoffatom oder durch mindestens eine Sulfonylgruppe

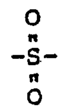

unterbrochen sein, und

n eine Zahl von 2 bis 5 darstellen.

2. Di- und Oligo-1,2,4-triazolidin-3,5-dione der allgemeinen Formel (I) gemäß Anspruch 1, dadurch gekennzeichnet, daß $R^1$ einen der nachstehenden Reste bedeutet:

1. $-(CH_2)_n-$ n = 2-20,

2. $-[CH_2-CH(R^2)-O]_m-CH_2-CH(R^2)-$ $R^2$ = H, $CH_3$; m = 1-9

3. $-[CH_2-(CH_2)_q-N(R_3)]_p-(CH_2)_q-CH_2-$ $R_3$ = Alkyl $C_1$-$C_4$, p = 1-4, q = 1-2;

4. 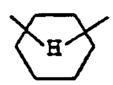

5. 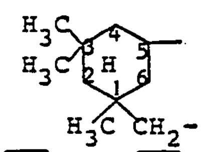

6. 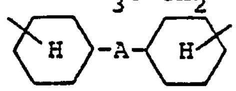 A = Alkylen mit 1-4 C-Atomen, O, —N($CH_3$)—;

7. 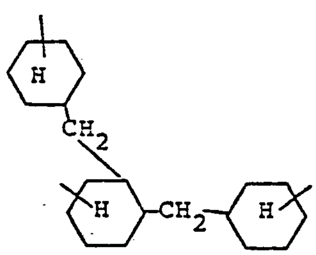

8. 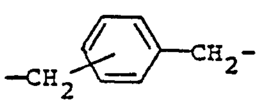

9. 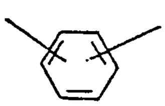

10. 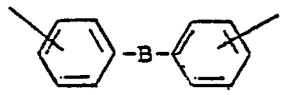 B = Alkylen mit 1-4 C-Atomen, O, $-N(CH_3)-$,

11. 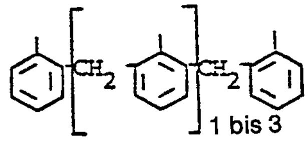

12. $-(CH_2)_3-O\{CH_2\}_{2\ bis\ 4}-O-(CH_2)_3-$

3. Verfahren zur Herstellung der Verbindungen der Formel (I) gemäß Anspruch 1, dadurch gekennzeichnet, daß man 0,9 bis 1,1 Mol Hydrazodicarbonamid oder 0,9 bis 1,1 Mol 1,2,4-Triazolidin-3,5-dion mit 1/m Mol eines Amins (m = Anzahl der $NH_2$-Gruppen pro Mol Amin) der Formel

$$R^1(NH_2)_n,$$

worin

$R^1$ und n die in Formel (I) des Anspruchs 1 angegebene Bedeutung besitzen, in Gegenwart oder Abwesenheit eines Lösungsmittels oder Lösungsmittelgemisches bei Temperaturen von 150 bis 280 °C und Drucken von 50 mbar bis 5 bar, gegebenenfalls in Gegenwart eines sauren oder basischen Katalysators unter Abspaltung von Ammoniak zu den Verbindungen der Formel (I) umsetzt.

4. Verfahren zur Herstellung der Verbindungen der Formel (I) gemäß Anspruch 1, dadurch gekennzeichnet, daß man ein monosubstituiertes Hydrazodicarbonamid der Formel

$$(H_2N-CO-NH-NH-CO-NH)_nR^1$$

worin

$R^1$ und n die in Anspruch 1 angegebene Bedeutung haben, in Gegenwart oder Abwesenheit eines Lösungsmittels oder Lösungsmittelgemisches bei Temperaturen von 150 bis 280 °C und Drucken von 50 mbar bis 5 bar, gegebenenfalls in Gegenwart eines sauren oder basischen Katalysators unter Abspaltung von Ammoniak zu den Verbindungen der Formel (I) umsetzt.

**Claims**

1. Di- and oligo-1,2,4-triazolidine-3,5-diones of the general formula (I),

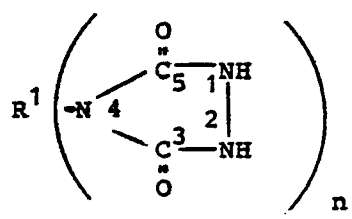 (I)

wherein

$R^1$ denotes a di- to penta-functional unsubstituted or substituted, linear or branched aliphatic $C_2$-$C_{30}$ radical, a di- to penta-functional unsubstituted or substituted cycloaliphatic $C_5$-$C_{21}$ radical, a di- to penta-functional unsubstituted or substituted aliphatic-aromatic $C_7$-$C_{17}$ radical or a di- to penta-functional unsubstituted or substituted aromatic $C_6$-$C_{21}$ radical, the afore-mentioned substituted radicals contain as substituents at least one alkoxycarbonyl group with $C_1$-$C_4$ in the alkoxy group, CN, $NO_2$, alkyl-mercapto group with $C_1$-$C_4$ in the alkyl group, dialkyl-amino group with $C_1$-$C_6$ in each alkyl group and halogen, the afore-mentioned aliphatic radicals can furthermore be interrupted by one or more oxygen atoms or tertiary nitrogen atoms, the afore-mentioned polynuclear aliphatic-aromatic, polynuclear cycloaliphatic and polynuclear aromatic radicals can be interrupted by at least one alkylene group with 1-4 C atoms, by at least one oxygen atom or tertiary nitrogen atom or by at least one sulphonyl group

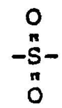

and

n represents a number from 2 to 5.

2. Di- and oligo-1,2,4-triazolidine-3,5-diones of the general formula (I) according to Claim 1, characterised in that $R^1$ denotes one of the following radicals :

1. $-(CH_2)_n-$ n = 2-20,

2. 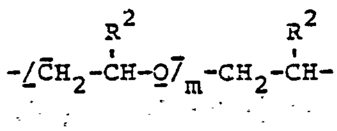 $R^2$ = H, $CH_3$ ; m = 1-9

3. 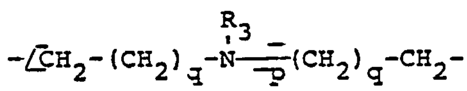 $R_3$ = alkyl $C_1$-$C_4$, p = 1-4, q = 1-2 ; ,

4. 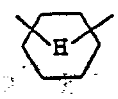

5. 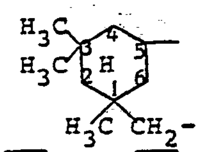

6. 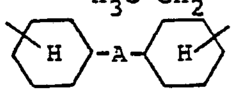 A = alkylene with 1-4 C atoms, O, —N($CH_3$)— ;

7. 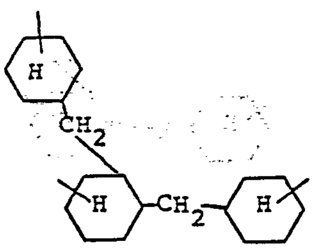

8. 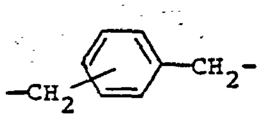

9. 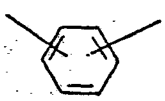

10. 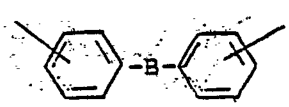 B = alkylene with 1-4 C atoms, O, —N($CH_3$)—,

11. 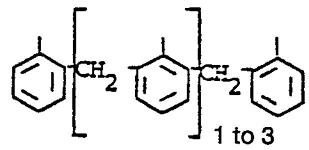

12. $-(CH_2)_3-O\{CH_2\}_{2\ to\ 4}-O-(CH_2)_3-$

3. Process for the preparation of the compounds of the formula (I) according to Claim 1, characterised in that 0.9 to 1.1 mol of hydrazodicarbonamide or 0.9 to 1.1 mol of 1,2,4-triazolidine-3,5-dione are reacted with 1/m mol of an amine (m = number of the $NH_2$ groups per mol of amine) of the formula

$$R^1(NH_2)_n,$$

wherein

$R^1$ and n have the meaning indicated in formula (I) of Claim 1, in the presence or absence of a solvent or solvent mixture at temperatures of 150 to 280 °C and pressures of 50 mbars to 5 bars, if appropriate in the presence of an acid or basic catalyst, with the elimination of ammonia, to give the compounds of the formula (I).

4. Process for the preparation of the componds of the formula (I) according to Claim 1, characterised in that a mono-substituted hydrazodicarbonamide of the formula

$$(H_2N—CO—NH—NH—CO—NH)_nR^1$$

wherein

$R^1$ and n have the meaning indicated in Claim 1, is reacted in the presence or absence of a solvent or solvent mixture at temperatures of 150 to 280 °C and pressures of 50 mbars to 5 bars, if appropriate in the presence of an acid or basic catalyst, with the elimination of ammonia, to give the compounds of the formula (I).

**Revendications**

1. Di- et oligo-1,2,4-triazolidine-3,5-diones de formule générale (I)

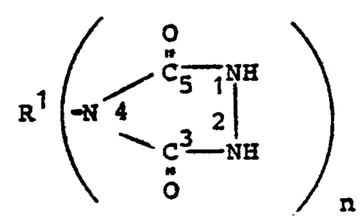 (I)

dans laquelle

$R^1$ représente un reste en $C_2$ à $C_{30}$ aliphatique linéaire ou ramifié, non substitué ou substitué, divalent à pentavalent, un reste en $C_5$ à $C_{21}$ cycloaliphatique non substitué ou substitué divalent à pentavalent, un reste en $C_7$ à $C_{17}$ aliphato-aromatique non substitué ou substitué divalent à pentavalent ou un reste en $C_6$-$C_{21}$ aromatique non substitué ou substitué divalent à pentavalent, les restes substitués mentionnés ci-dessus contiennent comme substituants au moins un groupe alkoxycarbonyle ayant de 1 à 4 atomes de carbone dans le groupe alkoxy, CN, $NO_2$, un groupe alkylmercapto ayant de 1 à 4 atomes de carbone dans le groupe alkyle, un groupe dialkylamino ayant de 1 à 6 atomes de carbone dans chaque groupe alkyle et un halogène, les restes aliphatiques mentionnés ci-dessus peuvent en outre être interrompus par un ou plusieurs atomes d'oxygène ou atomes d'azote tertiaire, les restes aliphato-aromatiques polynucléaires, cycloaliphatiques polynucléaires et aromatiques polynucléaires mentionnés ci-dessus pouvant être interrompus par au moins un groupe alkylène ayant de 1 à 4 atomes de carbone, par au moins un atome d'oxygène ou un atome d'azote tertiaire ou par au moins un groupe sulfonyle

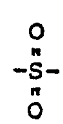

et

n est un nombre de 0 à 5.

2. Di- et oligo-1,2,4-triazolidine-3,5-diones de formule générale (I) suivant la revendication 1, caractérisées en ce que $R^1$ désigne l'un des restes suivants :

1. $-(CH_2)_n-$ n = 2-20,

2. 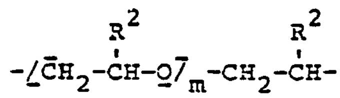 $R^2$ = H, $CH_3$ ; m = 1-9

3. 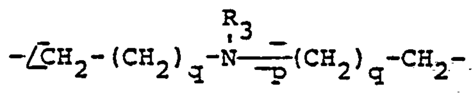 $R_3$ = Alkyle en $C_1$-$C_4$
p = 1-4,
q = 1-2 ;

4. 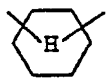

5. 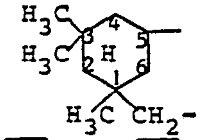

6. 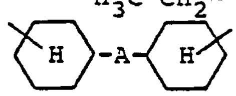 A = Alkylène avec 1-4 atomes de carbone, O, —N($CH_3$)— ;

7. 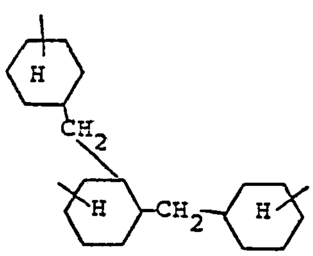

8. 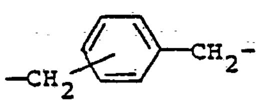

9. 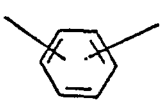

10. 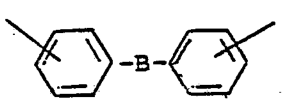 B = Alkylène avec 1-4 atomes de carbone, O, —N($CH_3$)—,

11. 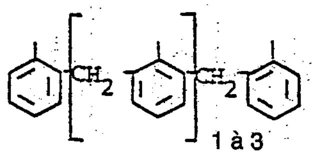

12. $-(CH_2)_3-O\{CH_2\}_{2\text{ à }4}-O-(CH_2)_3-$

3. Procédé de production des composés de formule (I) suivant la revendication 1, caractérisé en ce qu'on fait réagir 0,9 à 1,1 mole d'hydrazodicarboxamide ou 0,9 à 1,1 mole de 1,2,4-triazolidine-3,5-dione avec 1/m mole d'une amine (m = nombre de groupes $NH_2$ par mole d'amine) de formule

$$R^1(NH_2)_n,$$

dans laquelle

$R^1$ et n possèdent la définition indiquée dans la formule (I) de la revendication 1, en présence ou en l'absence d'un solvant ou d'un mélange de solvants à des températures de 150 à 280 °C et sous des pressions de 50 mbars à 5 bars, éventuellement en présence d'un catalyseur acide ou basique avec élimination d'ammoniac pour former les composés de formule (I).

4. Procédé de production des composés de formule (I) suivant la revendication 1, caractérisé en ce qu'on fait réagir un hydrazodicarboxamide monosubstitué de formule

$$(H_2N—CO—NH—NH—CO—NH)_nR^1$$

dans laquelle

$R^1$ et n ont la définition indiquée dans la revendication 1, en présence ou en l'absence d'un solvant ou d'un mélange de solvants à des températures de 150 à 280 °C et à des pressions de 50 mbars à 5 bars, éventuellement en présence d'un catalyseur acide ou basique avec élimination d'ammoniac pour former les composés de formule (I).